# EUROPEAN PATENT SPECIFICATION

(11) **EP 3 313 360 B1**
(45) Date of publication and mention of the grant of the patent: **17.08.2022**
(21) Application number: 16732717.0
(22) Date of filing: 23.06.2016
(51) Int. Cl.: A61K 8/35, A61K 8/65, A61K 8/73, A61Q 19/00, A61Q 19/08, A61K 8/11

(54) **FORMULATION**
FORMULIERUNG
FORMULATION

(30) Priority: 26.06.2015 GB 201511255
(43) Date of publication of application: 02.05.2018
(73) Proprietor: Colltec Laboratories Limited, London WC1H 8BU (GB)
(72) Inventor: HYLAND, David, Aylesbury Buckinghamshire HP22 4LW (GB); GHATORA, Pupinder Singh, Aylesbury Buckinghamshire HP22 4LW (GB)
(74) Representative: Henderson, Helen Lee
(86) International application number: PCT/GB2016/051886
(87) International publication number: WO 2016/207642

(56) References cited:
- WO-A1-2014/191056
- WO-A2-2014/041528
- CN-A- 104 398 395
- US-A1- 2011 160 137
- US-A1- 2014 080 773
- US-B1- 6 306 435
- Anonymous: "GNPD - Natural Collagen Boosting Supplement", , 1 October 2013 (2013-10-01), XP055294590, Retrieved from the Internet: URL:http://www.gnpd.com/sinatra/recordpage /2094678/from_search/KAqd3aCplN/?page=1 [retrieved on 2016-08-09]
- Anonymous: "GNPD - Skincare Capsules", , 1 December 2012 (2012-12-01), XP055294591, Retrieved from the Internet: URL:http://www.gnpd.com/sinatra/recordpage /1915296/from_search/eiOJji7xCP/?page=1 [retrieved on 2016-08-09]

## Description

### Field of Invention

The present invention relates to formulations comprising collagen, wherein the formulation is encapsulated in an enteric capsule and is substantially free from carriers and free from excipients, and processes for producing the same.

### Background to the Invention

Reducing levels of natural collagen and hyaluronic acid lie at the heart of the ageing process; which is compounded by UV damage.

Collagen is one of the most abundant group of proteins in the human body and is the major component (80%) of connective tissue. It provides structural support throughout the body where it is found in skin (70% of which is collagen), muscle, ligaments, tendons, cartilage and bones.

A key component of the skin's structure, collagen fibres provide the infrastructure for elastin, which maintains skin elasticity, and for hyaluronic acid to trap moisture. As people age the body's collagen production reduces, which can result in an increase of wrinkles or fine lines, and decreasing smoothness and suppleness of the skin. Indeed, after the age of 25, collagen can be lost at a rate of 1.5% per year.

However, these signs of ageing can be treated or slowed down if total body collagen levels can be increased. Since collagen cannot be absorbed through the skin, people turn to collagen supplementation. Unfortunately, traditional sources of collagen (bovine and porcine) are not considered to be that well absorbed. However, in recent times there has been an advancement in collagen supplementation with the emergence of collagen peptides (especially fish collagen peptides). Studies range from showing increased absorption after ingestion, to other positive efficacious markers of absorption such as the synthesis of new collagen fibres and positive effects on the skin as would be expected by an increase in collagen.

However, not all collagen supplements are effective. Due to the heat sensitive nature of collagen it is generally encapsulated in soft gel capsules, which require little to no heat to be used during the encapsulation process. Collagen and the collagen peptide in hydrolysed collagen is a protein. Once the protein comes into contact with stomach acid the collagen is broken down into its constituent amino acids and will no longer be clinically effective. Liquids, tablets and most capsules are broken down in the stomach in this way and, as a result, insufficient collagen is absorbed to counter the natural depletion of the protein. Conversely, most tablets and capsules if not completely destroyed in the stomach will pass out of the digestive system intact and therefore have no clinical effectiveness. WO2014/041528 relates to a composition for the preventative and/or curative treatment of cutaneous signs of ageing and in particular to an oral composition for such treatment. The composition comprises collagen, and a combination of Reactive Oxygen Species (ROS) scavengers/antioxidants selected from the group comprising Vitamin C, disulfides, Vitamin E, carotenoids and coenzyme Q10. The composition is encapsulated in gelatine capsules.

An object of the present invention is to provide an improved skin care supplement.

### Description

According to a first aspect of the present invention a formulation comprising collagen encapsulated in an enteric capsule is provided in accordance with claim 1. Preferably the formulation is substantially free from carriers and substantially free from excipients. The formulations of the invention can help to replenish lost collagen, thereby reducing the appearance of fine lines and wrinkles and helping to combat the ageing process. Preferably the formulation is in a form suitable for oral administration to a subject. The subject is preferably a mammal, more preferably a human.

Formulations of the invention are encapsulated in enteric capsules, which are a type of delayed release dosage form. Enteric capsules are resistant to stomach acid, allowing the formulation be released in the small intestine, which provides the largest surface area for absorption of the contents of the capsule. Collagen is known to be especially sensitive to the low pH conditions of the stomach and must survive through gastric and bile acids to reach the gastrointestinal tract to be most effective. In this way the enteric capsules lead to improved absorption of the collagen formulations of the present invention.

The majority of capsules used in the pharmaceutical industry are made from gelatine, i.e., the capsule shell is or comprises gelatine. However, hard gelatine capsules tend to have a relatively high moisture content (typically 13-16%w/w) and are therefore not suited for encapsulating hygroscopic substances, such as collagen. In an embodiment of the invention, the enteric capsule is a hydroxypropyl methylcellulose (HPMC) capsule. HPMC capsule shells used herein have a lower moisture content, generally 10% w/w or less, typically 4-6% w/w compared with gelatine capsules and are therefore more suited for encapsulating hygroscopic substances. Preferred capsule shells of the invention have a moisture content of 10% w/w or less, and more preferred capsules comprise or consist essentially of HPMC.

The collagen may be bovine, porcine or marine collagen and can be hydrolysed collagen, such as collagen peptide. In preferred embodiments of the invention the collagen is marine collagen, preferably hydrolysed marine collagen. Type I, II, III, IV or V collagen can be used in formulations of the invention. Preferably the formulation comprises type I collagen.

In preferred embodiments of the invention the collagen is a collagen peptide, such as a collagen peptide having an average molecular weight of 2,000 Da or less. This can result improved absorption of the collagen in the small intestine.

Formulations of the invention can comprise 50 - 600 mg collagen, preferably 100 - 400 mg, more preferably about 266 mg collagen. It will be understood by those skilled in the art that these quantities refer to the amount of collagen in each capsule.

Formulations of the invention may additionally comprise one or more additional active components selected from hyaluronic acid, natural astaxanthin, synthetic astaxanthin, Vitamin A, Vitamin C (ascorbic acid), Vitamin E (tocopherois, tocotrienois), beta carotene, lutein, selenium, silica, alpha lipoic acid, catechins, Coenzyme Q10 (CoQ10), polyphenolic antioxidants (resveratrol, flavonoids), and carotenoids (lycopene, carotenes, lutein). The formulations of the present invention comprise hyaluronic acid and natural astaxanthin.

Astaxanthin is a powerful, naturally occurring carotenoid pigment found in certain marine plants and animals. It is commonly recognised as being one of the most powerful antioxidants found in nature. Unlike many other types of antioxidant astaxanthin can never become a pro-oxidant in the body and will therefore never cause harmful oxidation. Due to its molecular structure astaxanthin is very effective against singlet oxygen. It has a powerful scavenging ability for lipid and free radicals, and effectively breaks peroxide chain reactions. Astaxanthin has been found to improve skin moisture levels, elasticity and smoothness, while reducing wrinkles, freckles and spots.

The structure of astaxanthin is similar to lutein and zeaxanthin, two antioxidants that have been shown to reduce the risk of cataracts. But, while similar, astaxanthin has a stronger antioxidant activity and UV-light protection effect, both of which point to it being an excellent supplement for eye health maintenance. The antioxidant properties of astaxanthin have also been shown to improve heart health, cellular health and the body's immune system as well as having natural anti-inflammatory properties.

Astaxanthin used in formulations of the present invention may be in the form of a powder, preferably a powder formed by micro encapsulation of astaxanthin rich oil. The astaxanthin rich oil can be micro encapsulated in modified starch. This prevents the astaxanthin from oxidising on contact with oxygen molecules in the air, while the oil provides the lipids necessary to maximise absorption.

Formulations of the invention can comprise 0.1 - 3 mg astaxanthin, preferably 0.8 - 2 mg, more preferably about 1.3 mg astaxanthin. It will be understood by those skilled in the art that these quantities refer to the amount of astaxanthin in each capsule.

Hyaluronic acid is a glycosaminoglycan found throughout the body of mammals. It is particularly prevalent in connective tissue, the synovial fluid around joints, the eyes, the heart, intervertebral spinal discs and the skin. Hyaluronic acid functions to transport nutrients to cells and remove toxins. It also acts as a cushioning agent, lubricating joints and facilitating water retention in the skin, which is especially important for skin care. Hyaluronic acid can absorb more than one thousand times its weight in water. By increasing the skin's moisture content hyaluronic acid can result in a dramatic improvement in the skin's appearance.

Hyaluronic acid is also heavily involved in promoting and enhancing the skin's collagen matrix. When skin is damaged by free radicals it becomes inflamed, with cells in the dermis producing progressively less hyaluronic acid, which leads to wrinkle formation. Hyaluronic acid levels also naturally decrease with age.

Formulations of the invention may comprise 5 - 30 mg hyaluronic acid, preferably 10 - 20 mg, more preferably about 16.6 mg hyaluronic acid. It will be understood by those skilled in the art that these quantities refer to the amount of hyaluronic acid in each capsule.

Hyaluronic acid used in formulations of the invention may have an average molecular weight of 100 kDa or less, preferably 50 kDa or less, more preferably 20 kDa or less. In preferred embodiments of the invention hyaluronic acid has an average molecular weight of about 20 kDa.

In a preferred embodiment of the invention the formulation is substantially free from carriers and free from excipients. Preferably the formulation does not contain any carriers or excipients, or any such components are present at de minimis amounts. Many conventional tablet or capsule supplement formulations contain carriers and excipients in the form of fillers, binders, colourings, disintegrants, diluents and many others. Excipients such as povidone, polyvinylpyrrolidone (PVP), cellulosics and modified starches can constitute up to 85% w/w of a tablet formulation. Some such formulations have been reported to pass through the digestive system unscathed or to get stuck in the digestive system altogether; neither disintegrating, nor passing through. In contrast, the carrier and excipient free formulations of the present invention are free from such problems, leading to better absorption of the active components. In embodiments of the invention the formulation does not contain calcium. More preferably the formulation does not comprise chelated calcium.

In embodiments of the invention collagen, optionally combined with other active ingredients as identified herein, may comprise at least 90% by weight of the contents of the enteric capsule, preferably at least 95% or at least 98% or at least 99% by weight of the contents of the capsule. Preferably collagen, combined with other active ingredients as identified herein, comprises at least 100% by weight of the contents of the enteric capsule.

The formulation may consist essentially of collagen, hyaluronic acid and astaxanthin. In other words, these substances can be encapsulated within the enteric capsule with no other components being present. Thus, collagen, hyaluronic acid and astaxanthin may comprise at least 98% or at least 99% by weight of the contents of the capsule. Preferably, collagen, hyaluronic acid and astaxanthin may comprise 100% by weight of the contents of the capsule.

Formulations of the present invention are particularly suitable for use as a skin care supplement. Preferably the formulations are suitable for oral administration, preferably taken before sleep. For maximal benefits at least one capsule, preferably at least two capsules, more preferably three capsules, should be taken before sleep and at least 1 hour, preferably at least 1.5 hours after eating.

The present invention also provides a process for preparing a formulation as described above, the process comprising: filling enteric capsule shells with collagen and one or more of hyaluronic acid, natural astaxanthin, synthetic astaxanthin, Vitamin A, Vitamin C (ascorbic acid), Vitamin E (tocopherois, tocotrienois), beta carotene, lutein, selenium, silica, alpha lipoic acid, catechins, Coenzyme Q10 (CoQ10), polyphenolic antioxidants (resveratrol, flavonoids), and carotenoids (lycopene, carotenes, lutein), with no other components being included; sealing the capsule shells; and cleaning the sealed capsules. The process is preferably carried out under positive pressure to ensure that hygroscopic components of the formulation are not exposed to moisture.

### Example 1: Making and encapsulating the formulation

Capsule filling and packaging is undertaken by contract manufacture.

The capsules are filled and bottled in an ISO Class 9 Pharmaceutical clean room, which means the air and temperature levels are automatically controlled with 20 complete air changes every hour. The entire production process from unpacking the raw materials to bottling the capsules happens inside the clean room to maintain product quality.

The steps undertaken in the clean room are:
1. Raw material testing.
2. Raw material mixing - sourced active ingredients are mixed in the required volume for the formula without any additions.
3. Capsule filling - the sourced capsule shells are then filled with the formula powder, using a Trufil^{™} approach, and sealed.
4. Capsule cleaning - the capsules are tumble cleaned to remove any excess powder.
5. Capsule inspection.
6. Bottle filling - the capsules are then bottled and the bottles sealed with neck seals.

Final packaging and final quality control then takes place outside the clean room environment.

Once filled, each capsule contains 1.3 mg astaxanthin, 16.7 mg hyaluronic acid and 266 mg collagen.

The capsules do not contain any ingredients other than the active ingredients and thereby avoid problems associated with the presence of fillers, binders, excipients, colourings, disintegrates and diluents etc., which can be detrimental to absorption of the active ingredients.

### Capsule Shells

Capsule shells (DRcaps^{®}) are sourced from Capsugel^{®}.

Protecting active ingredients which are sensitive to the active environment of the stomach can be difficult. Often a delivery system solution is used that requires significant investment and lengthy processes after encapsulation. The sourced capsules are an acid-resistant alternative that provides protection. These capsules, made from hydroxypropyl methycellulose (HPMC), are resistant to a low pH environment such as that found in the stomach.

The capsules also have a low moisture content, making them an ideal oral dosage container for moisture sensitive or hygroscopic food supplement ingredients; collagen peptides are highly hygroscopic.

### Marine Collagen Peptide

Enzymatically hydrolysed marine collagen peptide with an average molecular weight of 2,000 Da (Peptan^{®} F2000HD) is sourced from Rousselot^{®}.

To ensure maximum absorption collagen peptides with an average molecular weight of 2,000 Da are used; molecules of this weight have been shown to be readily absorbed by the small intestine.

Raw materials for the collagen peptide come exclusively from fish from facilities registered by the European Union and deemed fit for human consumption by official veterinarian post mortem inspection. The manufacturing process consists of the following steps: extraction with hot water, filtration, concentration, enzymatic hydrolysis, sterilisation at 138°C, and spray drying.

### Astaxanthin

Encapsulated natural astaxanthin (AstaReal^{®} EL25) is sourced from AstaReal^{®}.

There are two major problems in using astaxanthin in conjunction with a collagen peptide. These are:
1. Astaxanthin is exceptionally unstable when subjected to oxygen. Because it is such a strong antioxidant, the astaxanthin molecule will begin to bond with oxygen molecules in the air. Once it oxidizes, it will break down into a degradation product called "astacene" that has no benefit. Extreme care must therefore be taken when handling astaxanthin to be sure that it does not oxidize.
2. It is also highly recommended that astaxanthin is taken with meals, preferably with a meal that has some fat content. This is similar to other carotenoids, all of which are fat soluble. These lipophilic ("fat-loving") nutrients, taken in the absence of fats, are poorly absorbed; when taken with fats, the absorption is maximized. Collagen peptide absorption, on the other hand, is maximised if it is taken when the stomach is relatively empty.

These issues have been overcome by using a micro encapsulated astaxanthin rich oil with a significant lipid content; this approach both protects the astaxanthin molecule against oxidisation and provides the fat content required to maximise absorption.

The powder is manufactured by micro encapsulation of astaxanthin rich oil in modified starch. The astaxanthin is produced by non-solvent supercritical carbon dioxide extraction of crushed and spray dried aplanospores of the green microalga *Haematococcus pluvialis.*

### Hyaluronic Acid

Pharmaceutical quality hyaluronic acid with an average molecular weight of 20kDa is sourced from Ubichem.

The hyaluronic acid is manufactured by bacterial fermentation with a natural non-GMO strain, under GMP conditions and is of non-animal origin.

### Example 2

In 2008 an independent trial of Peptan^{®} F2000 Collagen Peptide in a non-enteric vegetable cellulose capsule was conducted by a third party, Ayton Online Research Limited, with a panel of 100 participants consisting of females between the ages of 35 and 65.

The trial involved each participant taking the recommended dose of 3 capsules nightly for a period of three months. Each participant was asked to complete a questionnaire before commencing the trial; at the end of months 1, 2, and 3; and on completion of the trial.

Peptan^{®} F2000 is a marine collagen peptide with an average molecular weight of 2,000 Da manufactured from the skin of Tilapia by enzymatic hydrolysis.

The questions and results from end of trial questionnaires were as follows:
1. How do you feel the product has improved the plumpness of your skin as compared to the start of the trial? Select score between 1 and 9 (1 = not at all, 9 = a lot)
   Over 89% of the participants indicated that they had noticed some improvement in the plumpness of their skin, with 72% scoring 5 or more against this question.
2. How do you feel the product has improved the firmness of your skin as compared to the start of the trial? Select score between 1 and 9 (1 = not at all, 9 = a lot)
   Over 86% of the participants indicated that they had noticed some improvement in the firmness of their skin, with 73% scoring 5 or more against this question.
3. How do you feel the product has improved the elasticity of your skin as compared to the start of the trial? Select score between 1 and 9 (1 = not at all, 9 = a lot)
   Over 85% of the participants indicated that they had noticed some improvement in the elasticity of their skin, with 67% scoring 5 or more against this question.
4. How effective was the product at smoothing the skin? Select score between 1 and 9 (1 = poor, 9 = good)
   Over 89% of the participants indicated that they had noticed some smoothing of their skin, with 68% scoring 5 or more against this question.
5. How effective was the product at repairing the skin? Select score between 1 and 9 (1 = poor, 9 = good)
   Over 88% of the participants indicated that the product improved skin repair, with 67% scoring 5 or more against this question.
6. How effective was the product at strengthening the skin? Select score between 1 and 9 (1 = poor, 9 = good)
   Over 88% of the participants indicated that they had noticed some strengthening of their skin, with 69% scoring 5 or more against this question.
7. How effective was the product at brightening the skin? Select score between 1 and 9 (1 = poor, 9 = good)
   Over 89% of the participants indicated that they had noticed some brightening of their skin, with 70% scoring 5 or more against this question.
8. How do you feel the product has improved the appearance of your skin tone as compared to before the trial? Select score between 1 and 9 (1 = not at all, 9 = a lot)
   Over 86% of the participants indicated that they had noticed some improvement in skin tone, with 71% scoring 5 or more against this question.
9. How do you feel the product has improved the youthfulness of your skin as compared to the start of the trial? Select score between 1 and 9 (1 = not at all, 9 = a lot)
   Over 85% of the participants indicated that they had noticed some improvement the youthfulness of their skin, with 66% scoring 5 or more against this question.
10. How effective was the product at strengthening your nails? Select score between 1 and 9 (1 = poor, 9 = good)
   Over 86% of the participants indicated that they had noticed some strengthening of their nails, with 68% scoring 5 or more against this question.
11. How effective was the product at strengthening your hair? Select score between 1 and 9 (1 = poor, 9 = good)
   Over 87% of the participants indicated that they had noticed some strengthening of their hair, with 66% scoring 5 or more against this question.
12. How effective was the product at improving the thickness of your hair? Select score between 1 and 9 (1 = poor, 9 = good)
   Over 84% of the participants indicated that they had noticed some thickening of their hair, with 62% scoring 5 or more against this question.
13. How do you feel the product has improved your sleep patterns as compared to before the trial? Select score between 1 and 9 (1 = not at all, 9 = a lot)
   Over 77% of the participants indicated that they had noticed some improvement in their sleep patterns, with 62% scoring 5 or more against this question.
14. How effective was the product at improving your body's vitality as compared to the start of the trial? Select score between 1 and 9 (1 = not at all, 9 = a lot)
   Over 80% of the participants indicated that they had noticed some improvement in their vitality, with 58% scoring 5 or more against this question.
15. How would you rate the product overall? Select score between 1 and 9 (1 = poor, 9 = good)
   The average rating for the product overall was 5.56. However, 63% of the participants scored the product at 5 or more.

## Claims

1. A formulation comprising collagen, hyaluronic acid and astaxanthin, wherein the formulation is encapsulated in an enteric capsule and wherein the collagen, hyaluronic acid and astaxanthin comprise at least 98% by weight of the contents of the capsule.

2. A formulation according to claim 1, wherein the enteric capsule is a hydroxypropyl methyl cellulose (HPMC) capsule.

3. A formulation according to claim 1 or 2, wherein the collagen is a collagen peptide having an average molecular weight of 2,000 Da or less.

4. A formulation according to any of claims 1 to 3, wherein the formulation comprises 50 - 600 mg collagen.

5. A formulation according to any of claims 1 to 4, wherein the formulation comprises 5 - 30 mg hyaluronic acid.

6. A formulation according any of claims 1 to 5, wherein the formulation comprises 0.1 - 3 mg astaxanthin.

7. A formulation according to any of claims 1 to 6, wherein the formulation is free from carriers, free from calcium, fillers, binders, colourings, disintegrants or diluents.

8. A formulation according to any of claims 1 to 7, for use as a skin care supplement.

9. A process for preparing a formulation according to any of claims 1 to 8, the process comprising:
filling enteric capsule shells with collagen, hyaluronic acid, and astaxanthin with no other components being included;
sealing the capsule shells; and
cleaning the sealed capsules.

10. A process according to claim 9, wherein the enteric capsule is a hydroxypropyl methyl cellulose (HPMC) capsule.

## Patentansprüche

1. Formulierung, umfassend Collagen, Hyaluronsäure und Astaxanthin, wobei die Formulierung in einer magensaftresistenten Kapsel eingekapselt ist und wobei das Collagen, die Hyaluronsäure und Astaxanthin wenigstens 98 Gewichtsprozent des Gehalts der Kapsel umfasst.

2. Formulierung nach Anspruch 1, wobei die magensaftresistente Kapsel eine Kapsel aus Hydroxypropylmethylcellulose (HPMC) ist.

3. Formulierung nach Anspruch 1 oder 2, wobei das Collagen ein Collagenpeptid mit einem mittleren Molekulargewicht von 2000 Da oder weniger ist.

4. Formulierung nach einem der Ansprüche 1 bis 3, wobei die Formulierung 50 bis 600 mg Collagen umfasst.

5. Formulierung nach einem der Ansprüche 1 bis 4, wobei die Formulierung 5 bis 30 mg Hyaluronsäure umfasst.

6. Formulierung nach einem der Ansprüche 1 bis 5, wobei die Formulierung 0,1 bis 3 mg Astaxanthin umfasst.

7. Formulierung nach einem der Ansprüche 1 bis 6, wobei die Formulierung frei von Trägern, frei von Calcium, Bindemitteln, Farbstoffen, Zerfallsmitteln oder Verdünnungsmitteln ist

8. Formulierung nach einem der Ansprüche 1 bis 7 zur Verwendung als ein Hautpflegeergänzungsmittel.

9. Verfahren zur Herstellung einer Formulierung nach einem der Ansprüche 1 bis 8, das Verfahren umfassend:
Füllen von magensaftresistenten Kapselhüllen mit Collagen, Hyaluronsäure und Astaxanthin, wobei keine anderen Komponenten enthalten sind,
Versiegeln der Kapselhüllen und
Reinigung der versiegelten Kapseln.

10. Verfahren nach Anspruch 9, wobei die magensaftresistente Kapsel eine Kapsel aus Hydroxypropylmethylcellulose (HPMC) ist.

## Revendications

1. Formulation comprenant du collagène, de l'acide hyaluronique et de l'astaxanthine, ladite formulation étant encapsulée dans une capsule entérique et dans laquelle le collagène, l'acide hyaluronique et l'astaxanthine comprennent au moins 98% en poids du contenu de la capsule.

2. Formulation suivant la revendication 1, dans laquelle la capsule entérique est une capsule en hydroxypropyle méthyle cellulose (HPMC).

3. Formulation suivant la revendication 1 ou 2, dans laquelle le collagène est un peptide de collagène ayant un poids moléculaire moyen de 2000 Da ou moins.

4. Formulation suivant la revendication 1 à 3, ladite formulation comprenant 50 à 600 mg de collagène.

5. Formulation suivant la revendication 1 à 4, ladite formulation comprenant 5 à 30 mg d'acide hyaluronique.

6. Formulation suivant la revendication 1 à 5, ladite formulation comprenant 0,1 à 3 mg d'astaxanthine.

7. Formulation suivant la revendication 1 à 6, ladite formulation étant libre de supports, de calcium, de charges, de liants, de colorants, de désintégrants ou de diluants.

8. Formulation suivant la revendication 1 à 7, ladite formulation étant destinée à une utilisation comme supplément de soin de peau.

9. Procédé de préparation d'une formulation suivant une des revendications 1 à 8, le procédé comprenant :
le remplissage d'enveloppes de capsules entériques avec du collagène, de l'acide hyaluronique et de l'astaxanthine sans inclure d'autres composants,
la fermeture hermétique des enveloppes de capsule, et
le nettoyage des capsules fermées hermétiquement.

10. Procédé suivant la revendication 9, dans lequel la capsule entérique est une capsule en hydroxypropyle méthyle cellulose (HPMC).
